# EUROPEAN PATENT APPLICATION

(11) **EP 2 676 619 A1**
(43) Date of publication of application: **25.12.2013**
(21) Application number: 13172212.6
(22) Date of filing: 17.06.2013
(51) Int. Cl.: A61B 17/34

(54) **Optical obturator assembly**

(30) Priority: 18.06.2012 US 201261660866 P; 06.05.2013 US 201313887420
(71) Applicant: Covidien LP, Mansfield, MA 02048 (US)
(72) Inventor: Fischvogt, Gregory, Hamden, CT Connecticut 06514 (US)
(74) Representative: Maschio, Antonio

(57) **Abstract**

An obturator assembly is presented including an obturator housing, an obturator shaft operably connected to the obturator housing and defining a longitudinal axis, and proximal and distal ends, and a penetrating member adjacent the distal end of the obturator shaft. The obturator assembly also includes an instrument retention mechanism mounted on a top portion of the obturator housing and adapted for securing and stabilizing surgical instruments, e.g., endoscopes, inserted into the obturator, thereby permitting visualization of tissue as the tissue is being penetrated by the penetrating member.

## Description

### CROSS REFERENCE TO RELATED APPLICATION

The present application claims the benefit of and priority to U.S. Provisional Application Serial No. 61/660,866, filed on June 18, 2012, the entire contents of which are incorporated herein by reference.

### BACKGROUND

### Technical Field

The present disclosure relates to an obturator assembly. More particularly, the present disclosure relates to a bladeless optical obturator assembly having an instrument retention mechanism mounted on an obturator housing.

### Background of Related Art

Minimally invasive procedures are continually increasing in number and variation. Forming a relatively small diameter, temporary pathway to the surgical site is a key feature of most minimally invasive surgical procedures. The most common method of providing such a pathway is by inserting a trocar assembly through the skin. Common trocar assemblies generally include an obturator assembly for penetrating the skin and a cannula assembly for providing a sealed passageway for insertion of surgical instruments into a body cavity. In many procedures, the trocar assembly is inserted into a body cavity of a patient and the body cavity is insufflated to provide a working space. Upon removal of the obturator assembly, the cannula assembly is utilized to provide the necessary pathway to the surgical site while minimizing leakage of insufflation gases. The obturator assembly may include a safety shield which protects against unintentional puncturing by a sharpened tip of the obturator assembly.

During certain particularly delicate operations, care is required to prevent underlying organs from being punctured by the sharpened tip of the obturator assembly. Therefore, it is desirable to provide an obturator assembly which includes safety mechanisms to prevent engagement of the sharpened tip of the obturator assembly with the underlying organs. It is further desirable to provide an obturator assembly having an obturator member and a bladeless penetrating member.

### SUMMARY

The present invention, according to various embodiments thereof, is directed to an obturator assembly for penetrating tissue and being at least partially positionable within a cannula assembly. The obturator assembly includes an obturator housing, an obturator shaft operably connected to the obturator housing and defining a longitudinal axis, and proximal and distal ends, and a penetrating member, e.g., which may be bladeless or sharp, adjacent the distal end of the obturator shaft. Additionally, the obturator assembly includes an instrument retention mechanism mounted on a top portion of the obturator housing and adapted for securing and stabilizing surgical instruments, e.g., endoscopes, inserted therethrough.

In further embodiments, the instrument retention mechanism may include an inner portion constructed from a first material having a first hardness value and an outer portion constructed from a second material having a second hardness value, the second hardness value being greater than the first hardness value. The inner portion of the instrument retention mechanism may include at least one ribbed protrusion configured to secure and stabilize the instrument retention mechanism to the top portion of the obturator housing.

In yet another embodiment, the instrument retention mechanism may include a pair of opposed extensions configured to facilitate securement of the instrument retention mechanism to the top portion of the obturator housing. The pair of opposed extensions may each include a rib member extending thereon.

Additionally, the instrument retention mechanism may include a centrally disposed opening for receiving the surgical instruments inserted therethrough, the opening including at least one pair of opposed slits adjacent to or in communication with the opening.

In yet another embodiment, the obturator shaft may include a plurality of equally spaced apart openings extending thereon.

Moreover, the bladeless penetrating member may define, from leading to trailing, a cylindrical element having a generally arcuate leading surface and a generally planar dissecting element extending from the cylindrical element. Alternatively, the penetrating member may define a dolphin-nose shape, e.g., having opposed concave surfaces circumferentially spaced between opposed convex surfaces.

In addition, the present invention, in various embodiments thereof, is directed to a trocar assembly for penetrating tissue. The trocar assembly includes a cannula assembly including a cannula housing and a cannula sleeve extending distally from the cannula housing and an obturator assembly, which may be an optical obturator assembly, at least partially positionable within the cannula assembly and being adapted to pass through tissue, the obturator assembly being removable from the cannula assembly subsequent to accessing an underlying tissue site. The obturator assembly may include an obturator housing, an obturator shaft connected to the obturator housing and defining a longitudinal axis, and proximal and distal ends, and a penetrating member, e.g., bladeless, adjacent the distal end of the obturator shaft. Additionally, the obturator assembly includes an instrument retention mechanism mounted on a top, e.g., proximal, portion of the obturator housing and adapted for securing and stabilizing surgical instruments inserted therethrough.

### BRIEF DESCRIPTION OF THE DRAWINGS

The accompanying drawings, which are incorporated in and constitute a part of this specification, illustrate embodiments of the disclosure and, together with a general description of the disclosure given above, and the detailed description of the embodiment(s) given below, serve to explain the principles of the disclosure, wherein:

FIG. 1 is a perspective view of a trocar assembly including an obturator assembly and a cannula assembly, in accordance with the present disclosure;

FIG. 2 is a perspective view of the trocar assembly of FIG. 1 illustrating the obturator assembly separated from the cannula assembly, in accordance with the present disclosure;

FIG. 3 is a perspective view of the obturator assembly, in accordance with the present disclosure;

FIG. 4A is a top, perspective view of the obturator assembly illustrating the instrument retention mechanism, in accordance with the present disclosure;

FIG. 4B is a top, perspective view of the instrument retention mechanism, in accordance with the present disclosure;

FIG. 4C is a bottom, perspective view of the instrument retention mechanism, in accordance with the present disclosure;

FIG. 5 is a side, cross-sectional view of a top portion of the obturator assembly, in accordance with the present disclosure;

FIG. 6 is a side, cross-sectional view of a top portion of the obturator assembly illustrating the pair of opposed extensions, in accordance with the present disclosure; and

FIG. 7 is a perspective view of a distal end of the obturator assembly illustrating the bladeless penetrating member, in accordance with the present disclosure.

The figures depict preferred embodiments of the present disclosure for purposes of illustration only. One skilled in the art will readily recognize from the following discussion that alternative embodiments of the structures and methods illustrated herein may be employed without departing from the principles of the present disclosure described herein.

### DETAILED DESCRIPTION

Embodiments of the presently disclosed apparatus will now be described in detail with reference to the drawings, in which like reference numerals designate identical or corresponding elements in each of the several views. As used herein, the term "distal" refers to that portion of the tool, or component thereof, which is further from the user while the term "proximal" refers to that portion of the tool or component thereof which is closer to the user.

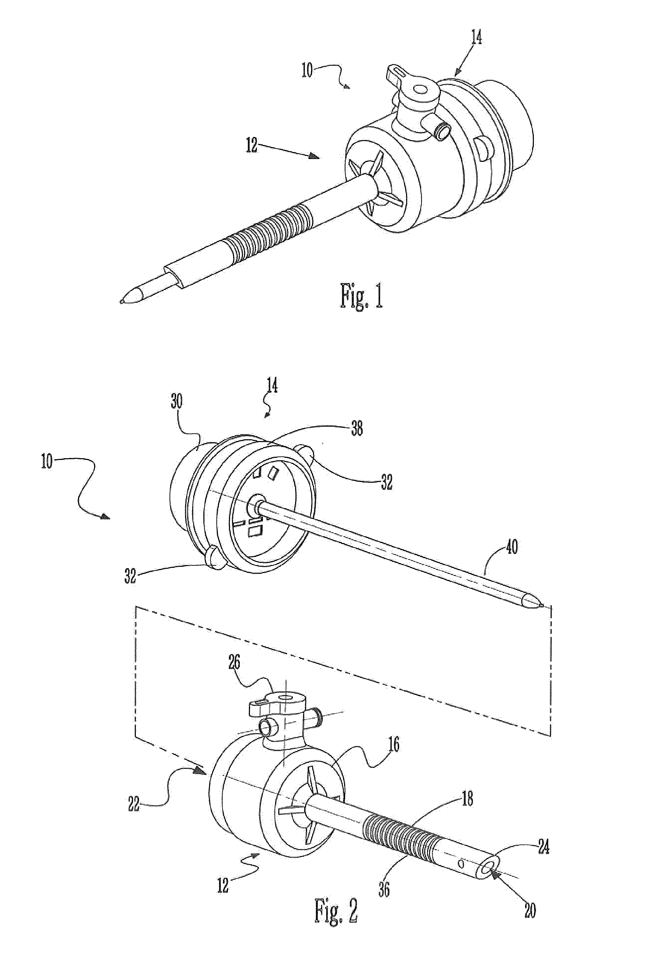

Referring to FIGS. 1 and 2, there is disclosed a trocar assembly 10 including a cannula assembly 12 and an obturator assembly 14 positioned through cannula assembly 12. Cannula assembly 12 provides an access port for various surgical instruments into the body as well as a conduit for a source of insufflation fluid to insufflate the body to create a working cavity.

With reference to FIG. 2, cannula assembly 12 includes a cannula housing 16 and elongate cannula sleeve 18 extending distally from cannula housing 16. Cannula housing 16 and cannula sleeve 18 define a throughbore 20, which extends from a proximal end 22 of cannula housing 16 to a distal end 24 of cannula sleeve 18. A valve 26 is provided on cannula housing 16 to receive a source of insufflation fluid for passage into the body of a patient. The cannula sleeve 18 may also include a plurality of ribs 36. The plurality of ribs 36 may be a plurality of protrusions or a plurality of recesses.

With reference to FIG. 2, obturator assembly 14 generally includes an obturator housing 38 and an elongate member 40 extending distally from, and movably mounted within, obturator housing 38. Obturator assembly 14 is additionally provided with a pair of wings 32 to facilitate securing cannula assembly 12 to the body of a patient. Obturator assembly 14 also includes an instrument retention mechanism 30, which is preferably releasably mounted to obturator housing 38. The instrument retention mechanism 30 is mounted on a top portion of the obturator housing 38 and adapted for securing and stabilizing surgical instruments, e.g., an endoscope, inserted therethrough. Methodologies for releasably connecting instrument retention mechanism 30 to obturator housing 38 may include a bayonet coupling, threaded connection, latch, friction fit, tongue and groove arrangements, snap-fit, etc. Preferably, obturator housing 38 is configured and dimensioned to functionally cooperate with cannulas that range in size, e.g., from about 5 mm to about 15 mm in diameter.

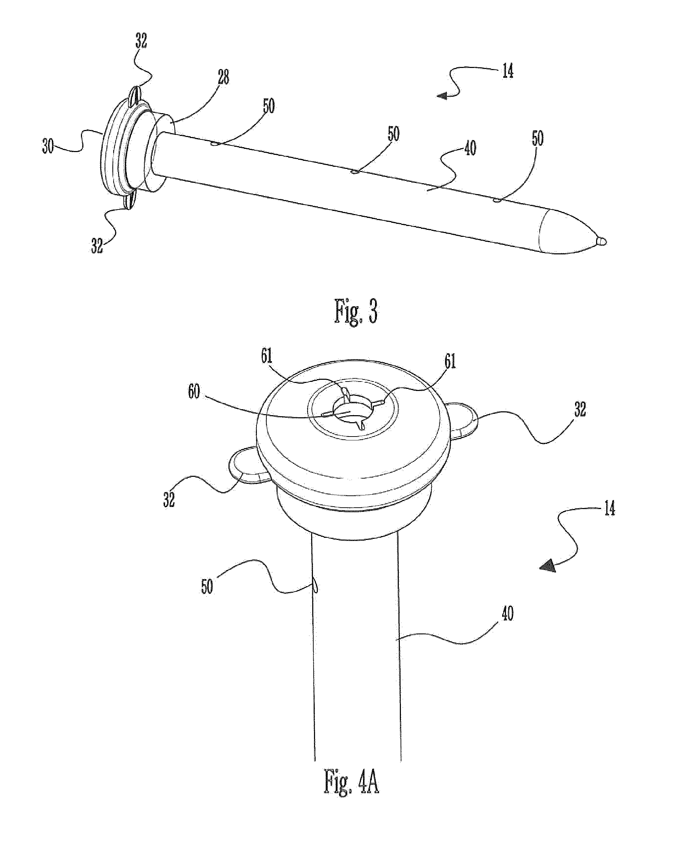

With reference to FIG. 3, a side view of the obturator assembly 14, in accordance with the present disclosure is presented. For sake of clarity, similar elements described with reference to FIGS. 1 and 2 will not be described with reference to FIG. 3. The side view of the obturator assembly 14 illustrates a plurality of openings 50 disposed across the length of the elongate member 40. The plurality of openings 50 may be formed by core support pins to prevent deflection of a core pin (not shown) during the manufacturing of the obturator assembly 14, as is well known in the art.

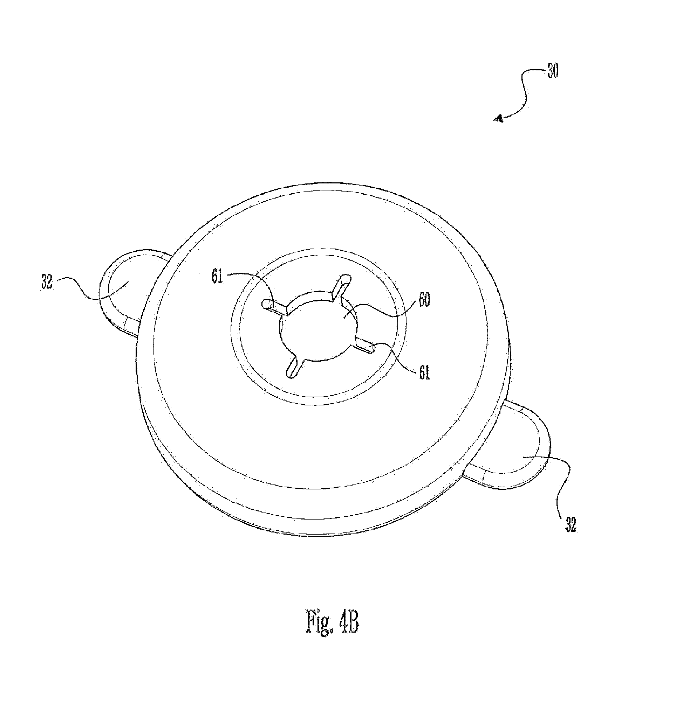

With reference to FIG. 4A, a perspective view of a top, perspective view of the obturator assembly 14 illustrating the instrument retention mechanism 30, in accordance with the present disclosure is presented. With reference to FIG. 4B, a top, perspective view of the instrument retention mechanism 30, in accordance with the present disclosure is presented. The instrument retention mechanism 30 includes an opening 60 having at least one pair of opposed slits 61. The opening 60 is configured to receive, for example, a surgical instrument therethrough. The opening 60, in conjunction with the slits 61, is dimensioned and adapted to releasably secure the one or more surgical instruments inserted therethrough.

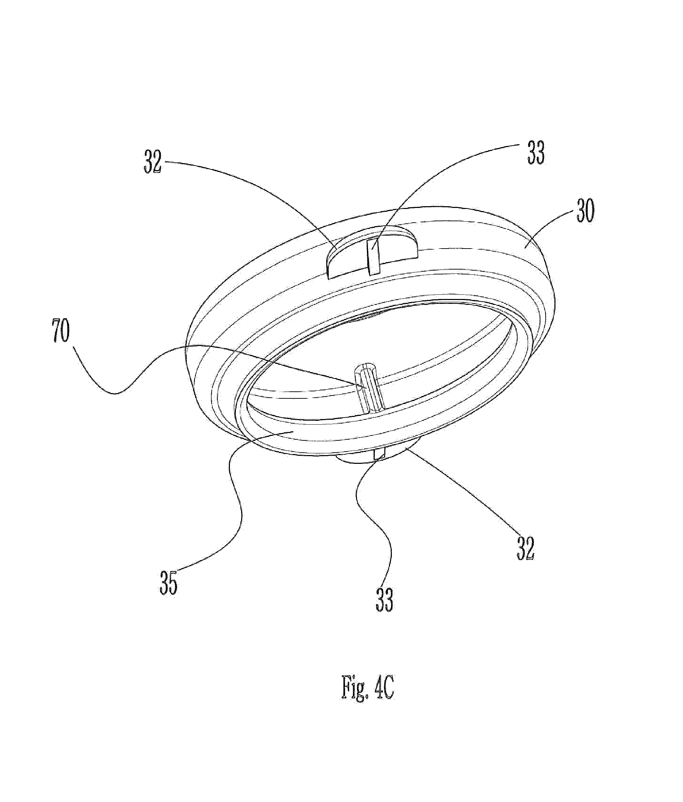

With reference to FIG. 4C, a bottom, perspective view of the instrument retention mechanism 30, in accordance with the present disclosure is presented. The bottom view illustrates that the wings 32 include at least one rib member 33 extending thereon. Additionally, the inner portion of the instrument retention mechanism 30 includes at least one ribbed protrusion 70 (or clocking rib) configured to secure and stabilize the instrument retention mechanism 30 to the top portion of the obturator housing 38. One skilled in the art may contemplate a plurality of ribbed protrusions 70 extending in an equally spaced apart manner across the inner surface of the instrument retention mechanism 30.

Moreover, the instrument retention mechanism 30, as shown in FIG. 4C, may include an inner portion constructed from a first material having a first hardness value and an outer portion constructed from a second material 35 having a second hardness value, the second hardness value being greater than the first hardness value. Therefore, one skilled in the art may contemplate portions of the instrument retention mechanism 30 being constructed from different materials having different hardness values in order to optimize the stabilization of the instrument retention mechanism 30 onto the obturator housing 38 and to optimize the stabilization of an instrument, e.g., endoscope, that is removably inserted into the instrument retention mechanism 30. It should also be noted that the hardness of the materials from which the instrument retention mechanism 30 is constructed may simultaneously be chosen so as to enable the instrument retention mechanism 30 to be removably connected onto the obturator housing 38 by a user, if desired.

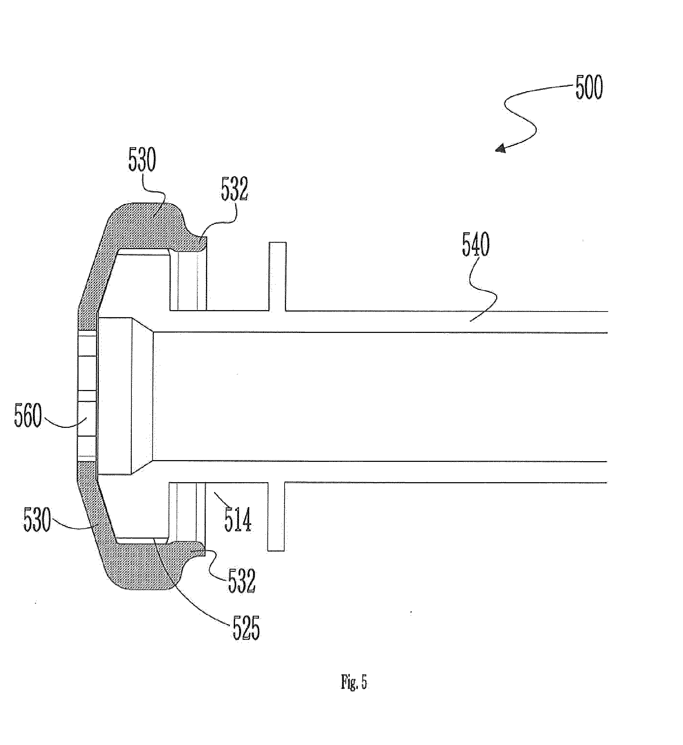

With reference to FIG. 5, a side, cross-sectional view 500 of a top portion of the obturator assembly 530, in accordance with the present disclosure is presented.

The cross-sectional view 500 depicts instrument retention mechanism 530 having a clamping mechanism 532 for being securedly fitted around the top portion of the obturator housing 514. The obturator housing 514 includes an obturator shaft 540 extending therefrom. The instrument retention mechanism 530 also includes a centrally disposed opening 560 for receiving at least one surgical instrument. The surface 525 of the obturator housing 514 is configured to encourage the instrument retention mechanism 530 to snap into place in the correct orientation. It is contemplated that the clamping mechanism 532 is constructed of a material having a greater hardness than the material used to construct the rest of the instrument retention mechanism 530.

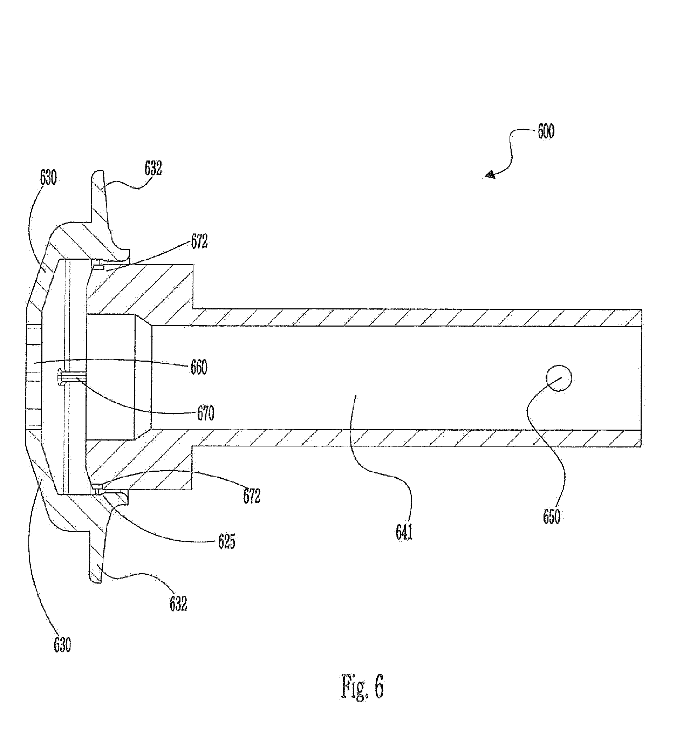

With reference to FIG. 6, a cross-sectional view 600 of a top portion of the obturator assembly illustrating the pair of opposed extensions 632, in accordance with the present disclosure is presented.

The cross-sectional view 600 illustrates the ribbed protrusion 670 (or clocking rib), which prevents internal geometry of the obturator assembly from being distorted or misaligned during the manufacturing process. The ribbed protrusion 670 may releasably cooperate with at least one recess 672 within the inner portions of the obturator assembly. The instrument retention mechanism 630 is also shown having a centrally disposed opening 660 for receiving at least one surgical instrument therethrough. The surface 625 of the obturator housing is configured to encourage the instrument retention mechanism 630 to snap into place in the correct orientation. It is contemplated that the clamping mechanism 632 is constructed of a material having a greater hardness than the material used to construct the rest of the instrument retention mechanism 630. The clamping mechanism 632 may be a pair of wings utilized to facilitate an assembler (not shown) to stretch and fit the instrument retention mechanism 630 to the obturator housing.

Moreover, an inner portion of the obturator shaft 641 may be hollow so as to permit the insertion of the one or more surgical instruments, e.g. an endoscope. Also, the obturator shaft 641 may include at least one opening 650. The opening 650 may be used as a core support to prevent deflection of a core pin (not shown) during the manufacturing of the obturator assembly.

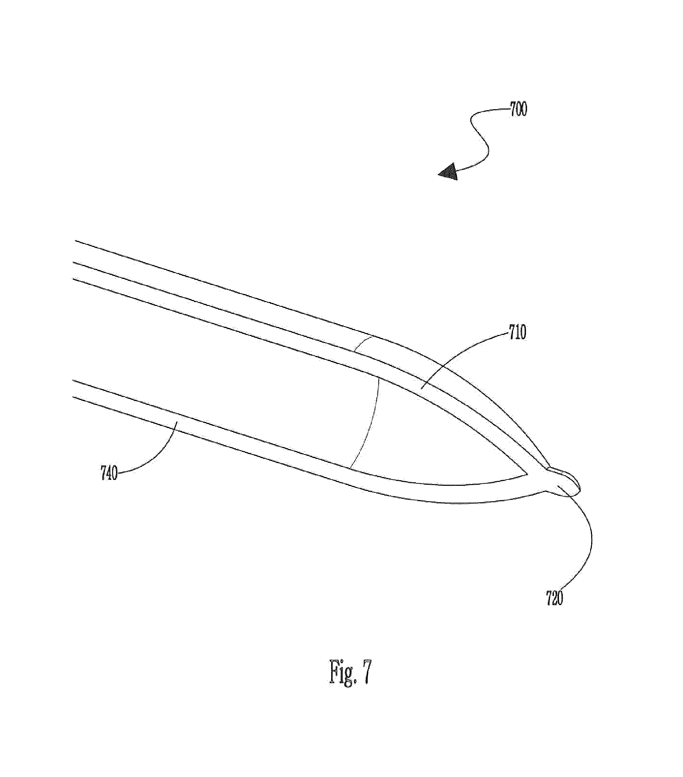

With reference to FIG. 7, a perspective view of a distal end 700 of the obturator assembly illustrating the penetrating member 710, in accordance with various embodiments of the present disclosure is presented. The penetrating member 710 may be bladeless, so as to provide for dissection through tissue planes/layers. Alternatively, the penetrating member 710 may have sharp edges so as to cut through tissue.

Penetrating member 710 may be tapered, conical, pyramidal, dolphin-nosed (as explained above), frusto-conical and/or any other configuration suitable for passing through tissue. In various embodiments, penetrating member 710 may, e.g., a dolphin-nose configuration as explained previously, with a rounded distal tip or nub 720 to facilitate initial penetration between tissue layers.

Penetrating member 710 may be substantially hollow to receive the distal end of, for example, an endoscope (not shown). Penetrating member 710 may be fabricated from any suitable plastic or polymeric material, e.g., polycarbonate, and advantageously is transparent or translucent to enable visualization therethrough. The penetrating member 710 may be either a separate component or integrally formed, e.g., monolithically, with elongate member 740. Elongate member 740 may be fabricated from the same transparent or translucent material, or alternatively the elongate member 740 may be fabricated from opaque material, such as metal or plastic.

Various components of the obturator assembly may include any suitable biocompatible metal, such as stainless steel and titanium and its alloys. Alternatively, the optical obturator assembly may include a polymeric material such as polycarbonate, polystyrene. Advantageously, elongate member 740 may be transparent throughout its entire length. Alternatively, only penetrating member 710, or even certain portions of the penetrating member 710, may be transparent or translucent.

Except where noted otherwise, the materials utilized in the components of the presently disclosed trocar assembly generally include materials such as, for example, ABS, polycarbonate, stainless steel, titanium and any other suitable biocompatible metals and/or polymeric materials. A preferred ABS material is CYCOLAC which is available from General Electric. A preferred polycarbonate material is also available from General Electric under the trademark LEXAN. An alternative polycarbonate material which may be utilized is CALIBRE polycarbonate available from Dow Chemical Company. The polycarbonate materials may be partially glass filled for added strength.

It will be understood that various modifications may be made to the embodiments disclosed herein. Therefore, the above description should not be construed as limiting, but merely as exemplifications of particular embodiments. Those skilled in the art will envision other modifications within the scope and spirit of the claims appended hereto.

The invention may be described by reference to the following numbered paragraphs:-
1. An obturator assembly for penetrating tissue and being at least partially positionable within a cannula assembly, the obturator assembly comprising:
   an obturator housing;
   an obturator shaft connected to the obturator housing and defining a longitudinal axis, and proximal and distal ends;
   a penetrating member adjacent the distal end of the obturator shaft; and
   an instrument retention mechanism mounted on the obturator housing and adapted for securing and stabilizing surgical instruments inserted therethrough.
2. The obturator assembly according to Paragraph 1, wherein the instrument retention mechanism includes an inner portion constructed from a first material having a first hardness value and an outer portion constructed from a second material having a second hardness value, the second hardness value being greater than the first hardness value.
3. The obturator assembly according to Paragraph 2, wherein the inner portion of the instrument retention mechanism includes at least one ribbed protrusion configured to secure and stabilize the instrument retention mechanism to the top portion of the obturator housing.
4. The obturator assembly according to Paragraph 1, wherein the instrument retention mechanism includes a pair of opposed extensions configured to facilitate securement of the instrument retention mechanism to the top portion of the obturator housing.
5. The obturator assembly according to Paragraph 1, wherein the penetrating member is bladeless.
6. The obturator assembly according to Paragraph 1, wherein the instrument retention mechanism includes a centrally disposed opening for receiving the surgical instruments inserted therethrough.
7. The obturator assembly according to Paragraph 6, wherein the opening including at least one pair of opposed slits adjacent to the opening.
8. The obturator assembly according to Paragraph 1, wherein the penetrating member defines a dolphin-nose shaped configuration.
9. A trocar assembly for penetrating tissue, the trocar assembly comprising:
   a cannula assembly including a cannula housing and a cannula sleeve extending distally from the cannula housing; and
   an optical obturator assembly at least partially positionable within the cannula assembly and being adapted to pass through tissue, the optical obturator assembly being removable from the cannula assembly subsequent to accessing an underlying tissue site, the optical obturator assembly including:
      an obturator housing;
      an obturator shaft connected to the obturator housing and defining a longitudinal axis, and proximal and distal ends;
      a penetrating member adjacent the distal end of the obturator shaft, at least a portion of the penetrating member permitting light to be transmitted therethrough; and
      an instrument retention mechanism mounted on a top portion of the obturator housing and adapted for securing and stabilizing an endoscope within the optical obturator for visualizing tissue as the tissue is penetrated by the penetrating member.
10. The trocar assembly according to Paragraph 9, wherein the cannula housing includes a valve positioned within an interior of the cannula housing
11. The trocar assembly according to Paragraph 9, wherein the instrument retention mechanism includes an inner portion constructed from a first material having a first hardness value and an outer portion constructed from a second material having a second hardness value, the second hardness value being greater than the first hardness value.
12. The trocar assembly according to Paragraph 11, wherein the inner portion of the instrument retention mechanism includes at least one ribbed protrusion configured to secure and stabilize the instrument retention mechanism to the top portion of the obturator housing.
13. The trocar assembly according to Paragraph 9, wherein the instrument retention mechanism includes a pair of opposed extensions configured to facilitate securement of the instrument retention mechanism to the top portion of the obturator housing.
14. The trocar assembly according to Paragraph 9, wherein the penetrating member is bladeless.
15. The trocar assembly according to Paragraph 9, wherein the instrument retention mechanism includes a centrally disposed opening for receiving the surgical instruments inserted therethrough.
16. The trocar assembly according to Paragraph 9, wherein the opening including at least one pair of opposed slits communicating therewith.
17. The trocar assembly according to Paragraph 9, wherein the penetrating member defines, from leading to trailing, a cylindrical element having a generally arcuate leading surface and a generally planar dissecting element extending from the cylindrical element.

## Claims

1. An obturator assembly for penetrating tissue and being at least partially positionable within a cannula assembly, the obturator assembly comprising:
an obturator housing;
an obturator shaft connected to the obturator housing and defining a longitudinal axis, and proximal and distal ends;
a penetrating member adjacent the distal end of the obturator shaft; and
an instrument retention mechanism mounted on the obturator housing and adapted for securing and stabilizing surgical instruments inserted therethrough.

2. The obturator assembly according to Claim 1, wherein the instrument retention mechanism includes an inner portion constructed from a first material having a first hardness value and an outer portion constructed from a second material having a second hardness value, the second hardness value being greater than the first hardness value.

3. The obturator assembly according to Claim 2, wherein the inner portion of the instrument retention mechanism includes at least one ribbed protrusion configured to secure and stabilize the instrument retention mechanism to the top portion of the obturator housing.

4. The obturator assembly according to any of Claims 1 to 3, wherein the instrument retention mechanism includes a pair of opposed extensions configured to facilitate securement of the instrument retention mechanism to the top portion of the obturator housing.

5. The obturator assembly according to any of Claims 1 to 4, wherein the penetrating member is bladeless.

6. The obturator assembly according to any of Claims 1 to 5, wherein the instrument retention mechanism includes a centrally disposed opening for receiving the surgical instruments inserted therethrough, preferably, wherein the opening including at least one pair of opposed slits adjacent to the opening.

7. The obturator assembly according to any of claims Claim 1 to 6, wherein the penetrating member defines a dolphin-nose shaped configuration.

8. A trocar assembly for penetrating tissue, the trocar assembly comprising:
a cannula assembly including a cannula housing and a cannula sleeve extending distally from the cannula housing; and
an optical obturator assembly at least partially positionable within the cannula assembly and being adapted to pass through tissue, the optical obturator assembly being removable from the cannula assembly subsequent to accessing an underlying tissue site, the optical obturator assembly including:
an obturator housing;
an obturator shaft connected to the obturator housing and defining a longitudinal axis, and proximal and distal ends;
a penetrating member adjacent the distal end of the obturator shaft, at least a portion of the penetrating member permitting light to be transmitted therethrough; and
an instrument retention mechanism mounted on a top portion of the obturator housing and adapted for securing and stabilizing an endoscope within the optical obturator for visualizing tissue as the tissue is penetrated by the penetrating member.

9. The trocar assembly according to Claim 8, wherein the cannula housing includes a valve positioned within an interior of the cannula housing

10. The trocar assembly according to Claim 8 or Claim 9, wherein the instrument retention mechanism includes an inner portion constructed from a first material having a first hardness value and an outer portion constructed from a second material having a second hardness value, the second hardness value being greater than the first hardness value, preferably, wherein the inner portion of the instrument retention mechanism includes at least one ribbed protrusion configured to secure and stabilize the instrument retention mechanism to the top portion of the obturator housing.

11. The trocar assembly according to any of Claims 8 to 10, wherein the instrument retention mechanism includes a pair of opposed extensions configured to facilitate securement of the instrument retention mechanism to the top portion of the obturator housing.

12. The trocar assembly according to any of Claims 8 to 11, wherein the penetrating member is bladeless.

13. The trocar assembly according to any of Claims 8 to 12, wherein the instrument retention mechanism includes a centrally disposed opening for receiving the surgical instruments inserted therethrough.

14. The trocar assembly according to any of Claims 8 to 13, wherein the opening including at least one pair of opposed slits communicating therewith.

15. The trocar assembly according to any of Claims 8 to 14, wherein the penetrating member defines, from leading to trailing, a cylindrical element having a generally arcuate leading surface and a generally planar dissecting element extending from the cylindrical element.
